# EUROPEAN PATENT SPECIFICATION

(11) **EP 0 518 696 B1**
(45) Date of publication and mention of the grant of the patent: **28.02.1996**
(21) Application number: 92305435.7
(22) Date of filing: 12.06.1992
(51) Int. Cl.: C07C 51/23, C07C 53/126, C07C 231/02, C08F 8/06, C08F 8/32

(54) **Process for preparing a polybutylcarboxylic acid**
Verfahren zur Herstellung einer Polybutylcarbonsäure
Procédé pour la fabrication d'un acide polybutylcarboxylique

(30) Priority: 14.06.1991 FR 9107699
(43) Date of publication of application: 16.12.1992
(73) Proprietor: BP Chemicals Limited, London EC2M 7BA (GB); BP CHEMICALS S.N.C., F-92400 Courbevoie (FR)
(72) Inventor: Blackborow, John Richard, BP Chemicals S.N.C., F-13117 Lavera (FR)
(74) Representative: Denbigh, Keith Warwick

(56) References cited:
- EP-A- 0 063 955
- BE-A- 737 825
- GB-A- 1 027 410
- US-A- 3 652 611
- US-A- 3 839 376
- PATENT ABSTRACTS OF JAPAN vol. 9, no. 283 (C-313)(2006) 9 November 1985 & JP-A-60 126 243

## Description

The present invention relates to a process for the production of a polybutylcarboxylic acid having a branched hydrocarbon chain. The obtained acid can be converted to an amide having a long hydrocarbon chain which can be used as an additive in lubricant oils or in fuels.

It is known to prepare long-chain carboxylic acids by conversion of a long-chain polyolefin. In particular, according to GB-B-1 027 410, it is known to convert a polyolefin to an acid by the addition of carbon monoxide and hydrogen,the so-called "OXO" reaction, followed by an oxidation reaction of the products obtained during the "OXO" reaction. This process has the disadvantage that an "OXO" reaction, generally takes place at high pressures and requires a cobalt-based catalyst which is difficult to use with polyolefins which can be relatively viscous. According to this patent, it is also known to convert a polyolefin to an acid by the addition of formaldehyde, the so-called "Prins" reaction, followed by oxidation of the products of the said reaction. This process has the major disadvantage that formaldehyde is a product difficult to use due to its susceptibility to polymerisation. Also according to this patent, it is known to convert a polyolefin to an acid by an addition of a halogen, followed by a hydrolysing oxidation reaction of the halogenated product obtained. However, this process leads to an acid which can contain traces of halogen, which are particularly troublesome, in particular if the acid or one of its derivatives is used in a lubricant or fuel composition.

According to US-A-3 652 611 a method is also known for directly converting 1,2-epoxide compounds to n-carboxylic acids providing a valuable end use for corresponding alpha-olefins. Straight chain epoxides are usefully employed in this process.

According to US-A-3 839 376 a process is also known for the catalytic oxidation of an epoxide. This epoxide is such that each of the contiguous carbon atoms of the heterocyclic epoxide ring is bound to at least one hydrogen atom. The oxidation process causes cleavage of the carbon to carbon bond of the contiguous carbon atoms to form two moles of same or different acids thereby sacrificing chain length.

A process has now been found which enables an acid to be obtained while avoiding the above-mentioned disadvantages. In particular, this process can be carried out at moderate pressures generally close to atmospheric pressure and enables an acid to be obtained which is free from halogen and which can be used, optionally in derivatised form, in lubricant compositions or in fuels.

The present invention therefore relates to a process for the production of a polybutylcarboxylic acid having a branched hydrocarbon chain, characterised in that a polybutylepoxide having branched hydrocarbon chain and containing from 36 to 360 carbon atoms is brought into contact with an oxidising compound, at a temperature sufficient to oxidise the polybutylepoxide to carboxylic acid.

According to the present invention, the polybutylepoxide used contains an oxygen atom bonded to two consecutive carbon atoms to form a heterocyclic epoxide ring, and has a branched long chain. The polybutylepoxide has from 36 to 360 and more particularly from 40 to 180 carbon atoms.

The polybutylepoxide is preformed by epoxidation of a polybutene. The latter can be a polybutene prepared by polymerisation of but-1-ene or of cis- or trans-but-2-ene, or by copolymerisation of but-1-ene with cis- or trans-but-2-ene, in the presence of a cationic catalyst. The polybutene may also be prepared by polymerisation of isobutene or by copolymerisation of isobutene with but-1-ene and/or cis- or trans-but-2-ene, in the presence of a catalyst of the cationic type, in particular a catalyst of the Friedel-Crafts type, such as aluminium trichloride, boron trifluoride, titanium tetrachloride or tin tetrachloride, or a chlorinated organoaluminium compound, optionally in the presence of a cocatalyst such as hydrochloric acid, tert-butyl chloride or water. The polybutene can advantageously be prepared by copolymerisation of a mixture of olefins having 4 carbon atoms and, in particular, a mixture containing 30 to 90% by weight of isobutene and 10 to 70% by weight of but-1-ene and but-2-enes.

The polybutene used for the preparation of the epoxide suitably contains one carbon-carbon double bond per molecule, arranged at the end of the chain.

This carbon-carbon double bond can be trisubstituted in accordance with the following formulae: 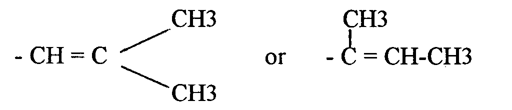
or tetrasubstituted in accordance with the formula: 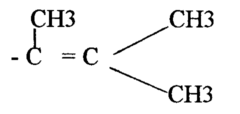
It can also be disubstituted of the vinylidene type, in accordance with the formula: 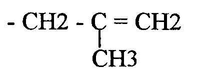
When a polybutene having one of the above formulae is used, a polybutylepoxide is formed in which at least one carbon atom of the heterocyclic ring is not bound to hydrogen atoms.

At least some molecules of the polybutene used for the preparation of the epoxide comprise vinylidene terminal double bonds eg from 1 to 90%, preferably from 40 to 85% with respect to all of the carbon carbon double bonds. In addition, it can have a weight-average molecular mass, determined by vapor pressure osmometry or by gel permeation chromatography, ranging from 200 to 10,000, preferably from 330 to 5,000 and more particularly from 400 to 2,500. Thus, the polybutene chain in the polybutylepoxide has from 9 to 90 and more particularly from 10 to 45 C4 hydrocarbyl units. Consequently, it contains from 36 to 360 carbon atoms and more particularly from 40 to 180 carbon atoms. It can have a viscosity, measured at 100°C, ranging from 0.002 to 100 Pa.s, preferably from 0.01 to 20 Pa.s and more particularly from 0.05 to 5 Pa.s. Moreover,the polybutylepoxide can have a relatively narrow molecular mass distribution. This distribution, calculated from the ratio of the weight-average molecular mass, Mw, to the number-average molecular mass, Mn, can range from 1 to 5 and preferably from 1 to 3. The polybutylepoxide is a liquid or a viscous product.

The epoxide used according to the invention can be prepared by all of the known methods and, in particular, by the method described in published EP-A-0 385 039.

The polybutylcarboxylic acid is produced by bringing the polybutylepoxide into contact with an oxidising compound.The oxidising compound can be chosen from a large number of compounds. Typically ,best results are obtained when the oxidising compound is nitric acid, oxygen, or ozone which can be used as a mixture with air or oxygen. This compound is generally used in a large molar excess relative to the polybutylepoxide and in particular with a molar ratio between the oxidising compound and the polybutylepoxide of greater than 1 and preferably greater than 2. However, if ozone is used, it is essential to ensure that the ozone is used under pressure and temperature conditions which prevent the formation of explosive mixtures. These conditions are very well known to those skilled in the art.

The polybutylepoxide is generally brought into contact with the oxidising compound by mixing the polybutylepoxide with the oxidising compound or, if the oxidising compound is a gas, by bubbling the oxidising gas through the polybutylepoxide. In this case, the oxidising gas is most often diluted with another gas, such as nitrogen, or in a mixture of gases, such as air. The oxidation generally takes place in the presence of a solvent, which can be a hydrocarbon, which is preferably aliphatic.The oxidation takes place at a temperature which can vary within a wide range depending on the nature of the oxidising compound used. This temperature can range from 60 to 150°C if nitric acid is used, from -50 to +20°C if ozone diluted in a gas is used, and from 100 to 230°C if atmospheric oxygen is used. The oxidation is suitably performed under atmospheric pressure. The oxidation can also take place in the presence of a catalyst. The latter can be a conventional acid catalyst, such as an alumina, an inorganic acid, a known oxidation catalyst, such as a cobalt salt or a manganese salt, or an inorganic acid anhydride.

This production of long-chain acid, most often gives a high yield of the polybutylcarboxylic acid, which is a surprising result in view of the fact that the polybutylepoxide used has a branched alkyl chain around the epoxide ring and has generally a high viscosity which is similar to the vicosity of the original polybutene. Furthermore this result is also surprising since the polybutylepoxide is not derived from a single alpha-olefin,but from a polymer having several types of carbon carbon double bonds.

The long-chain acid previously obtained according to the invention can be subsequently converted to a long-chain amide by a process in which the long-chain acid is reacted with a primary or secondary amine at a temperature sufficient to achieve a condensation reaction between the acid and the amine.

In this process, the amine is suitably an organic compound containing at least one primary or secondary amino functional group. This amine can be a monoamine, diamine or polyamine and can contain polyalkylene groups or heterocyclic groups. The amines most often used are chosen from a group comprising diethylenetriamine, di(methylethylene)triamine, triethylenetetramine, tri(methylethylene)tetramine, tri(ethylethylene)tetramine, tetraethylenepentamine, pentaethylenehexamine, ethylenediamine, hexamethylenediamine, ortho-phenylenediamine, meta-phenylenediamine, para-phenylenediamine, o-, m- and p-phenylenediamines substituted by at least one alkyl group, diamethylaminomethylamine, dimethylaminoethylamine, dimethylaminopropylamine, dimethylaminobutylamine, dimethylaminoheptylamine, diethylaminomethylamine, diethylaminopropylamine, diethylaminoamylamine, dipropylaminopropylamine, methylpropylaminoamylamine, propylbutylaminoethylamine, dimethylenetrianilinemethylenedianiline , polymethyleneaniline, polyalkylmethyleneaniline, morpholine, pyridine, piperidine, pyrrole, pyrimidine, pyrroline, pyrazine, pyridazine, N-(2-hydroxyethyl)ethylenediamine and the polyethers containing terminal amino functional groups and having the general formula

H2N (CH2 - CH2 - O)n CH2 - CH2 - NH2

where n is an integer ranging in general from 1 to 10.

In general, the long-chain acid and the amine are reacted under atmospheric pressure and at a temperature which is relatively high and can range from 50 to 250°C and preferably from 100 to 240°C. This reaction can be effected in the presence of a conventional straight-chain or cyclic hydrocarbon solvent, which can have from 5 to 20 carbon atoms. In general, in order to improve the amine yield, the solvent is so chosen as to form an azeotrope with water formed as by-product of the reaction, and the conditions of the reaction are such that the water is removed from the reaction mixture by means of azeotropic distillation. The reaction is generally stopped when no further water is removed from the reaction mixture. It is found that the reaction can take from 1 to 20 hours and preferably from 2 to 6 hours.

Moreover, the reaction is generally effected using a molar excess of amine The product resulting from the reaction is generally washed thoroughly with water in order to remove the unused amine. On the other hand, it can also be heated in order to remove any volatile components formed during the reaction and any solvent present which has been used.

The amide so formed can be used as an additive in lubricant oils or in fuels.

The following examples illustrate the present invention.

### Example 1:

### Preparation of a polybutylepoxide

700 ml of dichloromethane, as solvent, and 0.4 mol of a high vinylidene (75%) polybutene ULTRAVIS 10 (Registred Trade Mark) having an average molecular mass of 1017 g, were introduced into a 3 litre glass round-bottomed flask provided with a stirrer rotating at a speed of 200 revolutions per minute and a reflux column. 130 g of purified meta-chloroperbenzoic acid, diluted in 700 ml of dichloromethane, were then also added to the rection mixture, in the course of 1 hour, so as to maintain a gentle reflux of dichloromethane. Following this addition, the reaction mixture was continuously stirred for 4 hours, with dichloromethane reflux. At the end of this time, the reaction mixture was cooled to a temperature of -50°C with the aid of a mixture of solid carbon dioxide and acetone, so as to precipitate the meta-chlorobenzoic acid formed and the unreacted metachloroperbenzoic acid. The precipited solid products were then removed by filtration.The filtered mixture so formed was placed in a rotary evaporator so as to remove all of the dichloromethane used and 384 g of polybutylepoxide was thus obtained, which was then purified. For this purpose, the polybutylepoxide was mixed with 500 ml of n-hexane so as to obtain a solution. This solution was cooled to -50°C, filtered and then washed again, at ambient temperature, twice with 100 ml of an aqueous solution containing 10% by weight of sodium sulphite and three times with 200 ml of an aqueous solution containing 5% by weight of sodium bicarbonate. The organic hexane phase was then dried using calcium chloride and transferred to a rotary evaporator so as to remove all of the n-hexane used. Under these conditions, a purified polybutylepoxide (A) was obtained. Its infrared spectrum showed that it was totally free from carbon-carbon double bonds of the vinylidene type.

### Preparation of a polybutylcarboxylic acid

125 g of purified polybutylepoxide (A), 10 g of acidic alumina sold by MERCK under the reference 1078 and 20 mg of cobalt acetate were introduced into a 3 litre glass round-bottomed flask provided with a stirrer rotating at a speed of 200 revolutions per minute and a reflux column. The reaction mixture obtained was heated at 200°C, while passing through it a gas mixture containing 6% by volume of oxygen and 94% by volume of nitrogen, for 16 hours.

At the end of this time, the reaction mixture was cooled, then diluted with 100 ml of n-octane and then washed three times with 50 ml of water. It was then transferred into a rotary evaporator in order to remove all of the volatile components present and, in particular, the n-octane. Under these conditions, a polybutylcarboxylic acid (B) was obtained.

### Preparation of a polybutylamide

61.5 g of polybutylcarboxylic acid (B) and 13 g of N-(2-hydroxyethyl)ethylenediamine were introduced into a 0.25 litre glass round-bottomed flask provided with a stirrer rotating at 200 revolutions per minute and a reflux column.

The reaction mixture thus obtained was kept at 230°C for 10 hours. It was then cooled to ambient temperature, diluted with 100 ml of n-heptane and finally washed three times with 200 ml of water. The organic phase obtained after washing was transferred into a rotary evaporator in order to remove all of the volatile products present and in particular the n-heptane. Under these conditions, a polybutylamide (C) containing 1% by weight of nitrogen was obtained.

### Example 2:

### Preparation of a polybutylcarboxylic acid

40 g of purified polybutylepoxide (A), 10 ml of n-octane and 10 g of 50% nitric acid were introduced successively into a 0.25 litre glass round-bottomed flask provided with a stirrer rotating at a speed of 200 revolutions per minute and a reflux column. The reaction mixture obtained was heated under reflux at 110°C for 30 minutes. At the end of this time, this reaction mixture is cooled and was then diluted with 50 ml of water. After these washings,the reaction mixture was placed in a rotary evaporator so as to remove the volatile products present and in particular the n-octane. Under these conditions a polybutylcarboxylic acid (D) was obtained.

### Preparation of a polybutylamide

129 g of a polybutylcarboxylic acid (D) and 26 g of N-(2-hydroxyethyl)ethylenediamine were introduced into a 0.5 litre glass round-bottomed flask provided with a stirrer rotating at 200 revolutions per minute and a reflux column The reaction mixture thus obtained was kept at 230°C for 17 hours. It was then cooled to ambient temperature, diluted with 150 ml of n-octane and finally washed three times with 200 ml of water. The organic hydrocarbon phase obtained after washing was transferred into a rotary evaporator in order to remove all of the volatile products present and in particular the n-octane. Under these conditions a polybutylamide (E) containing 2.3% by weight of nitrogen was obtained.

## Claims

1. A process for the production of a polybutylcarboxylic acid having a branched hydrocarbon chain, characterised in that a polybutylepoxide having a branched hydrocarbon chain and containing from 36 to 360 carbon atoms is brought into contact with an oxidising compound, at a temperature sufficient to oxidise the polybutylepoxide to the corresponding carboxylic acid.

2. A process according to claim 1 characterised in that the oxidising compound is nitric acid.

3. A process according to claim 1 characterised in that the oxidising compound is oxygen.

4. A process according to claim 1 characterised in that the oxidising compound is ozone.

5. A process according to any one of claims 1 to 4 characterised in that the molar ratio of the oxidising compound to the polybutylepoxide is greater than 1.

6. A process according to any one of claims 1 to 5 characterised in that the oxidation of the polybutylepoxide takes place in the presence of a catalyst.

7. A process according to any one of claims 1 to 6 characterised in that the polybutylepoxide is obtained from a corresponding polybutene comprising from 1 to 90% of vinylidene terminal double bounds.

8. Use of the polybutylcarboxylic acid obtained by a process according to any one of the claims 1 to 7 in a process in which the polybutylcarboxylic acid is reacted with a primary or secondary amine to form a polybutenylamide.

## Patentansprüche

1. Verfahren zur Herstellung einer Polybutylcarbonsäure mit einer verzweigten Kohlenstoffwasserkette, dadurch gekennzeichnet, daß ein Polybutylepoxid mit einer verzweigten Kohlenwasserstoffkette und 36 bis 360 Kohlenstoffatomen bei einer zur Oxidation des Polybutylepoxids zu der entsprechenden Carbonsäure ausreichenden Temperatur mit einer oxidierenden Verbindung in Berührung gebracht wird.

2. Verfahren nach Anspruch 1, dadurch gekennzeichnet, daß die oxidierende Verbindung Salpetersäure ist.

3. Verfahren nach Anspruch 1, dadurch gekennzeichnet, daß die oxidierende Verbindung Sauerstoff ist.

4. Verfahren nach Anspruch 1, dadurch gekennzeichnet, daß die oxidierende Verbindung Ozon ist.

5. Verfahren nach einem der Ansprüche 1 bis 4, dadurch gekennzeichnet, daß das Molverhältnis oxidierende Verbindung/Polybutylepoxid größer als 1 ist.

6. Verfahren nach einem der Ansprüche 1 bis 5, dadurch gekennzeichnet, daß die Oxidation des Polybutylepoxids in Gegenwart eines Katalysators stattfindet.

7. Verfahren nach einem der Ansprüche 1 bis 6, dadurch gekennzeichnet, daß das Polybutylepoxid aus einem entsprechenden, 1 bis 90% terminale Vinylidendoppelbindungen umfassenden Polybuten erhalten wird.

8. Verwendung der nach einem Verfahren gemäß einem der Ansprüche 1 bis 7 erhaltenen Polybutylcarbonsäure bei einem Verfahren, bei dem die Polybutylcarbonsäure mit einem primären oder sekundärem Amin unter Bildung eines Polybutenylamids umgesetzt wird.

## Revendications

1. Procédé pour la production d'un acide polybutylcarboxylique ayant une chaîne hydrocarbonée ramifiée, caractérisé en ce qu'un polybutylépoxyde ayant une chaîne hydrocarbonée ramifiée et contenant de 36 à 360 atomes de carbone est mis en contact avec un oxydant, à une température suffisante pour oxyder le polybutylépoxyde en acide carboxylique correspondant.

2. Procédé selon la revendication 1, caractérisé en ce que l'oxydant est de l'acide nitrique.

3. Procédé selon la revendication 1, caractérisé en ce que l'oxydant est de l'oxygène.

4. Procédé selon la revendication 1, caractérisé en ce que l'oxydant est de l'ozone.

5. Procédé selon l'une quelconque des revendications 1 à 4 caractérisé en ce que le rapport molaire de l'oxydant au polybutylépoxyde est supérieur à 1.

6. Procédé selon l'une quelconque des revendications 1 à 5, caractérisé en ce que l'oxydation du polybutylépoxyde a lieu en présence d'un catalyseur.

7. Procédé selon l'une quelconque des revendications 1 à 6, caractérisé en ce que le polybutylépoxyde est obtenu à partir d'un polybutène correspondant comprenant de 1 à 90% de doubles liaisons terminales vinylidène.

8. Utilisation de l'acide polybutylcarboxylique obtenu par un procédé selon l'une quelconque des revendications 1 à 7 dans un procédé dans lequel on fait réagir l'acide polybutylcarboxylique avec une amine primaire ou secondaire pour former un polybuténylamide.
